(19) Europäisches Patentamt
European Patent Office
Office européen des brevets

(11) Veröffentlichungsnummer: **0 002 765**
**B1**

# (12) EUROPÄISCHE PATENTSCHRIFT

(45) Veröffentlichungstag der Patentschrift: **02.09.81**

(51) Int. Cl.³: **C 07 D 501/34,** A 61 K 31/545

(21) Anmeldenummer: **78101738.9**

(22) Anmeldetag: **16.12.78**

(54) **Cephalosporinderivate, Verfahren zu ihrer Herstellung und pharmazeutische Präparate sowie deren Herstellung.**

(30) Priorität: **24.12.77 DE 2758001**

(43) Veröffentlichungstag der Anmeldung: **11.07.79 Patentblatt 79/14**

(45) Bekanntmachung des Hinweises auf die Patenterteilung: **02.09.81 Patentblatt 81/35**

(84) Benannte Vertragsstaaten: **BE CH DE FR GB IT NL SE**

(56) Entgegenhaltungen: **FR-A-2 294 690**

(73) Patentinhaber: **HOECHST AKTIENGESELLSCHAFT, Postfach 80 03 20, D-6230 Frankfurt/Main 80 (DE)**

(72) Erfinder: **Blumbach, Jürgen, Dr., Rauenthaler Weg 18, D-6000 Frankfurt/Main (DE)**
Erfinder: **Dürckheimer, Walter, Dr., Im Lerchenfeld 45, D-6234 Hattersheim (DE)**
Erfinder: **Schrinner, Elmar, Dr., Hedwigstrasse 2, D-6200 Wiesbaden (DE)**

EP 0 002 765 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Cephalosporinderivate, Verfahren zu ihrer Herstellung und pharmazeutische Präparate sowie deren Herstellung

Gegenstand der Erfindung sind Cephalosporinderivate der allgemeinen Formel I

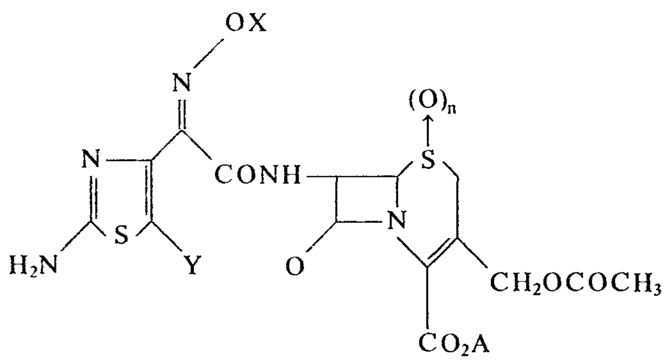

(I)

in der

n = 0 oder 1 und X Wasserstoff, Alkyl mit 1 bis 4 C-Atomen, Carboxymethyl, Alkoxycarbonylmethyl mit 1 bis 4 C-Atomen in der Alkoxygruppe, Aminocarbonylmethyl oder Cyanomethyl bedeuten, wobei die Carboxymethyl-, Alkoxycarbonylmethyl-, Aminocarbonylmethyl- und Cyanomethylgruppe an ihrer Methylengruppe auch noch durch Alkyl mit 1—4 C-Atomen substituiert sein können, Y für Methyl oder Halogen und A für Wasserstoff, einen leicht abspaltbaren Esterrest oder ein physiologisch unbedenkliches Kation steht und die Gruppe =N—OX in der syn-Form vorliegt.

Steht X für Alkyl mit 1 bis 4 C-Atomen, so seien insbesondere die Reste Methyl, Äthyl, Propyl und Butyl, vorzugsweise Methyl, genannt.

Steht X für Alkoxycarbonylmethyl mit 1 bis 4 C-Atomen im Alkylteil, so kommen insbesondere Methoxycarbonylmethyl und Äthoxycarbonylmethyl in Betracht.

X in der vorstehenden Bedeutung von Carboxymethyl, das auch in Form seiner physiologisch unbedenklichen Salze vorliegen kann, Alkoxycarbonylmethyl, Aminocarbonylmethyl und Cyanomethyl kann in der Methylengruppe substituiert sein durch Alkyl mit 1 bis 4 C-Atomen, vorzugsweise Methyl.

Im einzelnen seien als besonders bevorzugte Reste X genannt Wasserstoff, Methyl, Äthyl, n-Propyl, iso-Propyl, n-Butyl, iso-Butyl, Carboxymethyl, Methoxycarbonylmethyl, Äthoxycarbonylmethyl, Aminocarbonylmethyl und Cyanomethyl.

Für Y sind bevorzugte Reste: Methyl, Brom, Chlor und Fluor, besonders bevorzugt jedoch Chlor und Fluor.

Als leicht spaltbare Ester in der Bedeutung von A seien als bevorzugt erwähnt der tert.-Butylester und Trimethylsilylester, sowie auch der Benzyl-, Benzhydryl-, Trichloräthyl-, Benzoylmethyl-, Methoxymethyl- oder p-Methoxybenzylester.

Als physiologisch unbedenkliche Kationen A seien beispielsweise genannt ein Alkali-, insbesondere das Natrium- und Kaliumion, ein Erdalkali-, insbesondere das Calcium- und Magnesiumion und Ammoniumion, vorzugsweise jedoch ein Natriumion, sowie ein gegebenenfalls substituiertes alkyliertes Ammoniumion, wie vorzugsweise Triäthylammonium, Diäthylammonium, Dimethylammonium oder Morpholinium, Benzylammonium, Procainium, L-Argininium und L-Lysinium. Entsprechende physiologisch unbedenkliche Kationen kommen auch für den Fall in Betracht, daß X in Form eines Salzes der Carboxymethylgruppe vorliegt.

Die Hydroximino- bzw. Alkoximinogruppe in den Verbindungen der allgemeinen Formel I, III, IV und V liegt in der syn-Form vor. Die Bezeichnung syn bzw. anti drückt die räumliche Lage relativ zur Carboxyamidgruppe in den Verbindungen I und III zur Carboxylgruppe in den Verbindungen III und zur Alkoxycarbonylgruppe in den Verbindungen V aus, wobei die syn-Position diejenige ist, bei der die OX-Gruppe auf der gleichen Seite der C=N-Doppelbindung steht, wie die Carboxyamid-, Carboxyl- oder Alkoxycarbonylgruppe.

2-Aminothiazole der allgemeinen Formel I, III, IV und V können in jeweils zwei tautomeren Formen auftreten, die im Gleichgewichtszustand nebeneinander vorliegen und durch die folgenden Gleichgewichtsgleichungen dargestellt werden können.

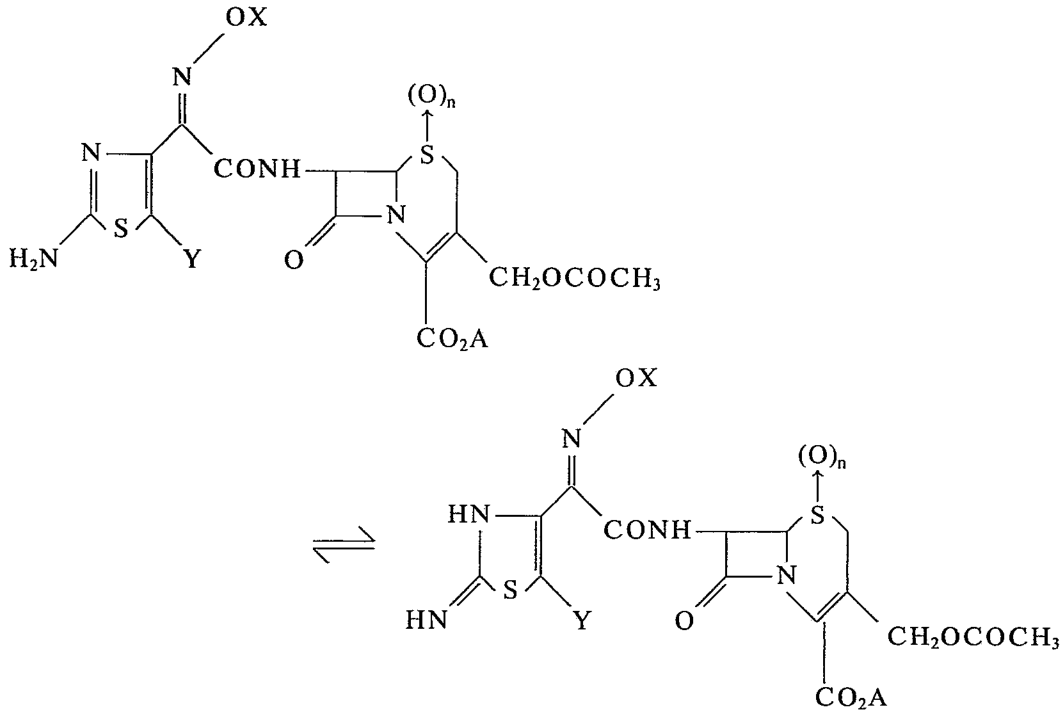

(I)

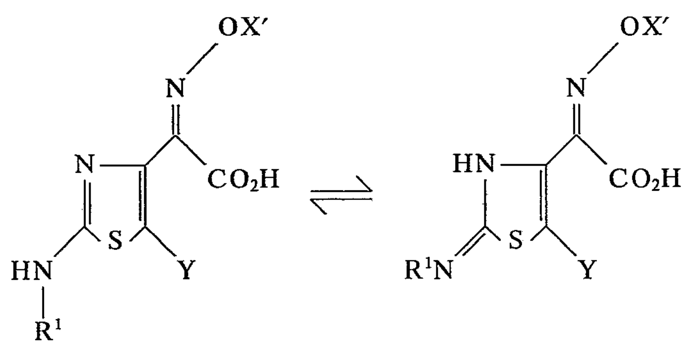

(III)

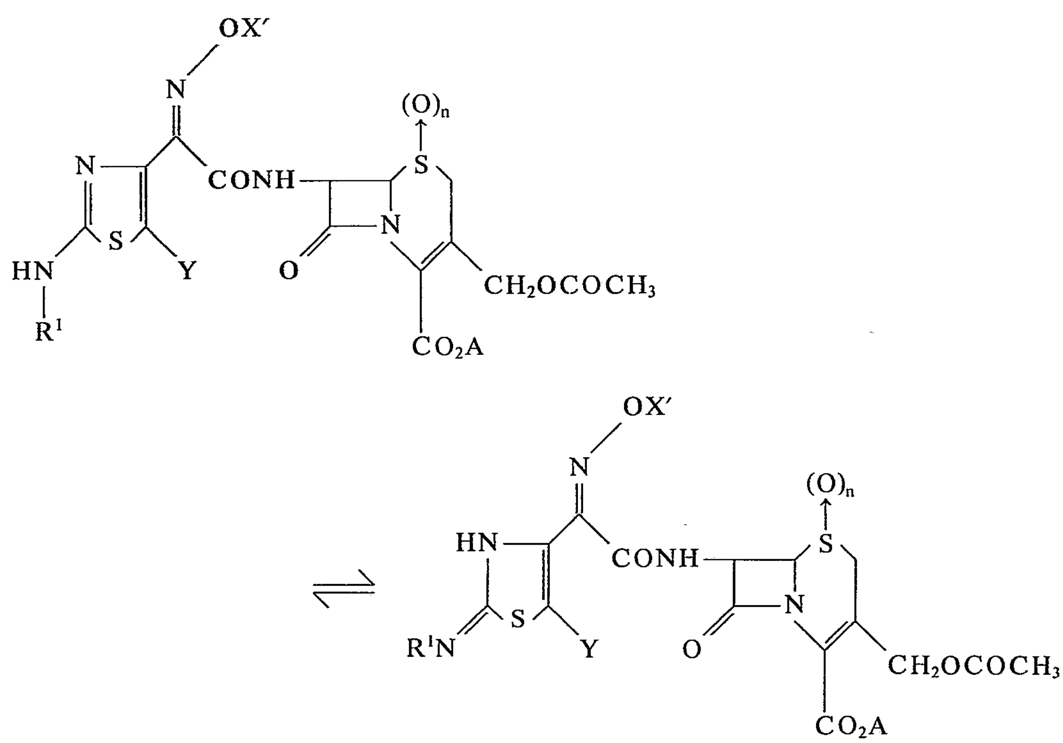

(IV)

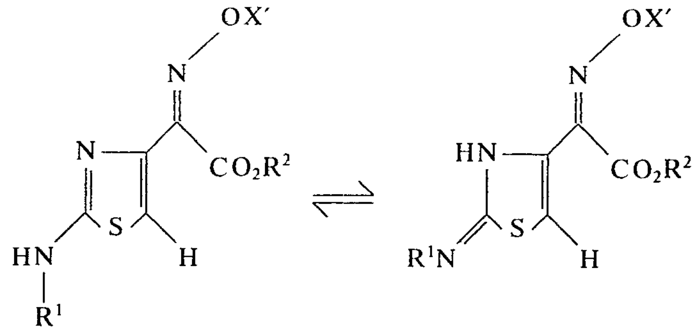

(V)

In den vorliegenden Unterlagen wird davon abgesehen, bei formelmäßigen Darstellungen jeweils beide Tautomeren wiederzugeben. Sie beschränken sich aus Zweckmäßigkeitsgründen auf die Wiedergabe des Amino-thiazol-tautomeren

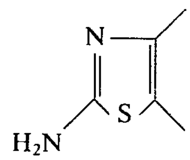

worauf sich auch die Nomenklatur der Verbindungen bezieht.

Gegenstand der Erfindung ist weiterhin ein Verfahren zur Herstellung von Cephemverbindungen der allgemeinen Formel I, das dadurch gekennzeichnet ist, daß man Lactame der allgemeinen Formel II

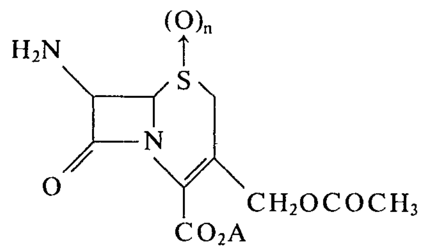

(II)

in der A und n die oben angegebenen Bedeutungen haben mit einer Carbonsäure der allgemeinen Formel III

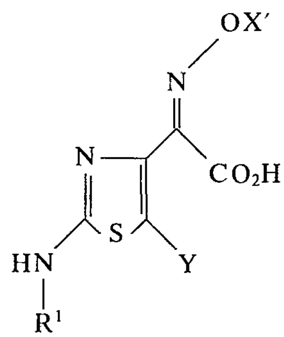

(III)

oder einem aktivierten Derivat derselben zu einer Verbindung der allgemeinen Formel IV

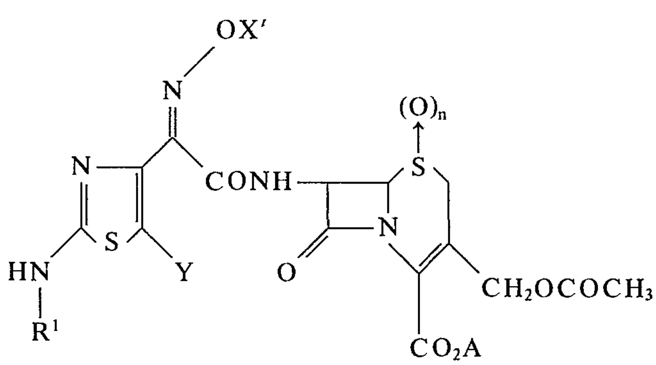

(IV)

umsetzt und

a) gegebenenfalls das so erhaltene Produkt am Schwefel des Cephemrings zum R- oder S-Sulfoxid oxidiert und/oder
b) einen Rest $R^1$ in der Bedeutung einer Schutzgruppe abspaltet und/oder
c) den Rest X', soweit er nicht die Bedeutung von X hat, in den Rest X überführt.

In den Formeln III und IV steht

$R^1$ für Wasserstoff oder für eine aus der Peptidchemie bekannte Amino-Schutzgruppe, wie z. B. gegebenenfalls substituiertes Alkyl, wie vorzugsweise tert.-Butyl, tert.-Pentyl, Benzyl, p-Methoxybenzyl, Benzhydryl, Trityl und Phenyläther, gegebenenfalls substituiertes aliphatisches Acyl, wie z. B. Formyl, Chloracetyl, Bromacetyl, Trichloracetyl und Trifluoracetyl oder gegebenenfalls substituiertes Alkoxycarbonyl, wie beispielsweise Trichloräthoxycarbonyl oder Benzyloxycarbonyl,
X' für X, für eine leicht abspaltbare Gruppe wie z. B. Formyl, Trifluoracetyl, Chloracetyl, Bromacetyl, Trityl, tert.-Pentyl, tert.-Butyl, Benzhydryl, Tetrahydropyranyl, vorzugsweise jedoch für tert.-Butyl, Trityl und Tetrahydropyranyl, sowie für eine Gruppe der Formel $-CH_2CO_2R^3$, in der $R^3$ die Bedeutung eines unter milden Bedingungen abspaltbaren Restes hat, wie vorzugsweise Trichloräthyl, tert.-Butyl, Benzyl, p-Methoxyphenyl, Benzhydryl oder Trityl.

Als aktivierte Derivate der Carbonsäuren der allgemeinen Formel III eignen sich insbesondere die Halogenide, vorzugsweise Chloride und Bromide, ferner die Anhydride und gemischten Anhydride, die Azide und aktivierten Ester, vorzugsweise die mit p-Nitrophenol, 2,4-Dinitrophenol, Methylencyanhydrin, N-Hydroxysuccinimid und N-Hydroxyphthalimid, besonders bevorzugt mit 1-Hydroxybenzotriazol und 6-Chlor-1-H-hydroxybenzotriazol. Als gemischte Anhydride eignen sich besonders solche mit niederen Alkansäuren, z. B. mit Essigsäure und besonders bevorzugt mit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren III, in denen $R^1$ nicht Wasserstoff bedeutet, mit Chlorameisensäurebenzylester, -p-nitrobenzylester, -iso-butylester, -äthylester oder -allylester gewinnt. Die aktivierten Derivate können als isolierte Substanzen, aber auch in situ umgesetzt werden. Allgemein erfolgt die Umsetzung der Cephemderivate II mit der Carbonsäure III oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z. B. Diäthyläther und vorzugsweise Tetrahydrofuran und Dioxan; Ketone, wie vorzugsweise Aceton und Butanon; Amide, wie vorzugsweise Dimethylformamid und Dimethylacetamid oder Wasser. Es kann sich auch als vorteilhaft erweisen, Gemische der genannten Lösungsmittel zu verwenden. Dies ist oftmals der Fall, wenn die Cephemverbindung II mit einem in situ erzeugten aktivierten Derivat der Carbonsäure III umgesetzt wird.

Die Umsetzung von Cephemverbindungen II mit Carbonsäuren III bzw. deren aktivierten Derivaten kann in einem Temperaturbereich von etwa $-50$ bis etwa $+80°C$, vorzugsweise zwischen $-20$ und $+50°C$, besonders bevorzugt jedoch zwischen $-20°C$ und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

In einzelnen Fällen kann es sich auch als vorteilhaft erweisen, die freie Carbonsäure der Formel III mit einer Cephemverbindung der Formel II, in der A die Bedeutung eines leicht abspaltbaren Esterrestes, wie vorzugsweise tert.-Butyl oder Trimethylsilyl, hat und n 0 und 1 sein kann, direkt umzusetzen, wobei man vorteilhaft ein wasserbindendes Mittel in annähernd äquimolarer Menge zusetzt. Als solches kommen zum Beispiel Carbodiimide in Frage, insbesondere Dicyclohexylcarbodiimid. Diese Reaktion wird in inerten Lösungsmitteln, wie vorzugsweise Methylenchlorid, Dimethylformamid, Tetrahydrofuran, Dioxan oder auch Gemischen durchgeführt.

Die Umsetzung von aktivierten Derivaten der Carbonsäuren der Formel III mit Cephemverbindungen der Formel II wird vorzugsweise in alkalischem Milieu oberhalb pH 7 vorgenommen. Man setzt hierfür dem Reaktionsgemisch eine Base zu, wie vorzugsweise Kalium- oder Natriumcarbonat, Kalium- oder Natriumbikarbonat, Kalium- oder Natriumhydroxyd, Pyridin oder ein Trialkylamin, wie z. B. Triäthylamin, N-Methylmorpholin, Äthyldiisopropylamin oder Kalium-tert-butylat.

Cephem-Verbindungen der Formel I können auch erhalten werden, indem der Rest $R^1$, sofern er nicht Wasserstoff bedeutet, in Verbindungen der Formel IV abgespalten und/oder der Rest X', sofern er nicht X bedeutet, in X umgewandelt wird.

So kann die Abspaltung von $R^1$ mit in der $\beta$-Lactam- und Peptid-Chemie allgemein üblichen schonenden Methoden, wie Hydrolyse in Säuren, vorzugsweise Ameisensäure oder Trifluoressigsäure, oder auch Hydrogenolyse in Gegenwart von Edelmetallkatalysatoren erfolgen. Je nach Schutzgruppe können aber auch spezielle Abspaltungsreagenzien zum Einsatz kommen, wie beispielsweise gegebenenfalls substituierte Thioharnstoffe zum Entfernen von $\alpha$-Halogenacylgruppen.

Auch die Umwandlung der Gruppe X', sofern sie nicht die Bedeutung von X hat, in X kann mit in der β-Lactam- und Peptidchemie üblichen schonenden hydrolytischen oder hydrogenolytischen Methoden herbeigeführt werden, wobei insbesondere Hydrolysen in anorganischen und organischen Säuren, wie vorzugsweise Trifluoressigsäure oder verdünnter Ameisensäure genannt sein sollen.

Bei der erfindungsgemäßen Umsetzung anfallende Ester der Formel I und IV, in denen A die Bedeutung eines leicht abspaltbaren Restes hat, können — falls gewünscht — in literaturbekannter, schonender Weise, z. B. hydrolytisch oder hydrogenolytisch, in Verbindungen der Formel I oder IV umgewandelt werden, in denen A die Bedeutung von Wasserstoff oder einem physiologisch unbedenklichen Kation hat, wie es vorstehend beschrieben worden ist. In analoger Weise kann auch aus dem Rest X' in der Bedeutung von $-CH_2CO_2R^3$ die $R^3$-Gruppe abgespalten werden.

Lactame der Formel II, in der n = 0 ist, können vorzugsweise als freie Säuren oder als Ester, jedoch auch als Salze oxidiert werden.

Vorteilhaft ist es, vorher die 7-Aminogruppe durch leicht abspaltbare Aminoschutzgruppen, wie sie beispielsweise in der Peptidchemie üblich sind, zu schützen. Als sauer abspaltbare Gruppen können beispielsweise eingesetzt werden: tert.-Butyl, Benzhydryl, tert-Butoxycarbonyl, Trityl, Benzyloxycarbonyl, 3,5-Dimethoxybenzyloxycarbonyl oder Trimethylsilyl. Bewährt hat sich auch der Schutz der Aminogruppe in Form einer sauer spaltbaren Schiffschen Base durch Umsetzung mit reaktiven Carbonylverbindungen, wie beispielsweise Benzaldehyd, Salicylaldehyd, p-Nitrobenzaldehyd, Furfurol, 5-Nitrofurfurol, Acetylaceton oder Acetessigester. Eine Spaltung der Schiffschen Base gelingt auch durch Umsetzung mit Hydrazin oder Phenylhydrazin, vorzugsweise mit Girard-Reagenz oder 2,4-Dinitrophenylhydrazin.

Zur Oxidation der Cephemverbindung II mit n = 0 eignen sich die literaturbekannten Methoden, die zur Bildung von Sulfoxiden durch Oxidation von Sulfiden führen (vgl. beispielsweise Methodicum Chimicum, Bd. 7 (1976) S. 693—698).

Als Oxidationsmittel haben sich z. B. bewährt Peroxyde, Hydroperoxyde, Persäuren, Wasserstoffsuperoxyd und deren Gemische mit anorganischen und organischen, oxidationsbeständigen Säuren wie beispielsweise Phosphorsäure, Ameisensäure, Essigsäure, Trifluoressigsäure. Die Persäuren können auch in situ durch Vermischen mit Wasserstoffperoxid erzeugt werden. 3-Chlorperbenzoesäure hat sich besonders bewährt. Sie wird vorteilhafterweise direkt eingesetzt.

Als Lösungsmittel für die Oxidation eignen sich alle unter den Reaktionsbedingungen stabilen Lösungsmittel, wie z. B. Dioxan, Tetrahydrofuran, Chloroform, Methylenchlorid, Essigsäure, Ameisensäure, Trifluoressigsäure, Benzol, Tetramethylharnstoff, Dimethylformamid oder Dimethylacetamid.

Die Menge des Oxidationsmittels beträgt mindestens 2 Oxidationsäquivalente (entsprechend einem aktiven Sauerstoffatom). Es kann jedoch auch ein geringer Überschuß in die Reaktion eingebracht werden.

Die Reaktionstemperatur liegt im Bereich zwischen etwa −20 und +80°C, vorzugsweise jedoch zwischen −20° und Raumtemperatur.

Die am Schwefel zum Sulfoxid aufoxidierten Cephemverbindungen II mit n = 1 können in der R- oder S-Konfiguration vorliegen.

(Zur Kennzeichnung der Konfiguration vgl. Angew. Chemie 78 [1966], S. 413).

Cephem-Verbindungen der Formel II mit n = 0, liefern bevorzugt die R-Sulfoxide, wenn die Aminogruppe in Form einer Schiffschen Base geschützt ist.

Acyl-Aminoschutzgruppen an der 7-Aminogruppe liefern überwiegend die 1-Sulfoxide mit der S-Konfiguration. Eine Unterscheidung und Trennung beider Konfigurations-Isomere gelingt chromatographisch. Auch die NMR-Spektroskopie kann zur Unterscheidung der R- und S-Sulfoxide herangezogen werden (vgl. E. H. Flynn, Cephalosporins and Penicillins, Chemistry and Biology, Academic Press, New York and London, 1972).

Verbindungen der Formel I und IV, in denen n = 1 ist, können auch erhalten werden, indem man die oben beschriebenen Stufen der Oxidation von Cephemverbindungen II zum Sulfoxid und die anschließende Acylierung mit einer Carbonsäure III oder einem aktivierten Derivat derselben vertauscht.

So kann man zunächst die Cephemverbindungen II, in denen n = 0 ist und A die obengenannten Bedeutungen hat, mit einer Carbonsäure III, in der $R^1$, X' und Y die obengenannten Bedeutungen haben, oder einem aktivierten Derivat derselben zu einer Verbindung der Formel IV, in der $R^1$, X', Y, A die obengenannten Bedeutungen haben und n = 0 ist, umsetzen. Die anschließende Oxidation zum Sulfoxid kann unter den für die Oxidation der Lactame der Formel II mit n = 0 in Lactamen der Formel II mit n = 1 beschriebenen Reaktionsbedingungen erfolgen. Ein Schutz der 7-Aminogruppe ist naturgemäß überflüssig, da durch die vorangegangene Acylierung mit den Carbonsäuren der Formel III die 7-Aminogruppe durch das Oxidationsmittel nicht mehr angegriffen wird. Die Oxidation der Verbindungen I und IV liefert vorwiegend Sulfoxide mit der S-Konfiguration, denen noch geringe Mengen Sulfoxide mit der R-Konfiguration beigemischt sein können, welche man in der oben beschriebenen Weise abtrennen kann.

Die zur Acylierung verwendeten Carbonsäuren III lassen sich nach verschiedenen Verfahren herstellen.

So erhält man beispielsweise Verbindungen der Formel III mit Y in der Bedeutung von Halogen, indem Verbindungen der Formel V

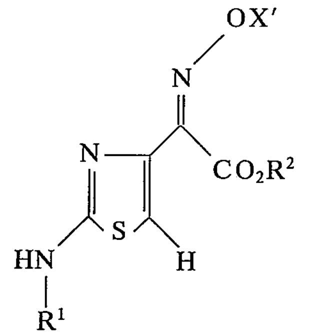

(V)

in der $R^1$ und X′ die obengenannten Bedeutungen haben und $R^2$ einen Alkylrest mit 1—4 C-Atomen oder einen Aralkylrest, wie vorzugsweise Benzyl- oder Phenyläthyl, bedeuten, mit einem halogenierenden Reagenz umgesetzt werden und gegebenenfalls der Rest $R^1$ und/oder der Rest X′ in die für die folgenden Reaktionen günstigste Form überführt wird und/oder der so erhaltene Ester V in die Carbonsäure mit der allgemeinen Formel III in an sich bekannter Weise überführt wird.

Als geeignete Halogenierungsmittel können die elementaren Halogene, wie bevorzugt Brom und Chlor, Trihalogen-isocyanursäuren, wie vorzugsweise Trichlorisocyanursäure, N-Halogenamide, wie vorzugsweise Chloramin-T, N-Chloracetamid, N-Brom-acetamid, N-Halogenimide, wie vorzugsweise N-Chlorsuccinimid, N-Bromsuccinimid, N-Chlorphthalimid, N-Bromphthalimid oder Alkylhypochloride, wie beispielsweise tert-Butylhypochlorid eingesetzt werden. Die Reaktion wird in der Regel in einem Lösungsmittel durchgeführt, das die Reaktion nicht nachteilig beeinflußt oder sie sogar in die gewünschte Richtung ablaufen läßt. So empfiehlt sich bei Alkylhypochloriten, N-Halogenamiden und N-Halogenimiden ein polares, hydroxylgruppenhaltiges Lösungsmittel, welches die Bildung von positiven Halogenionen fördert, so vorzugsweise Ameisensäure, Eisessig, Wasser, Alkanole wie z. B. Methanol, Äthanol oder Isopropanol. Daneben, vor allem aber bei der Verwendung von elementarem Halogen, können sich auch Lösungsmittel wie Chloroform, Methylenchlorid, Tetrahydrofuran, Dioxan oder Dimethylformamid oder Mischungen derselben untereinander oder mit den obengenannten hydroxylgruppenhaltigen Lösungsmitteln empfehlen.

Die Reaktionstemperatur ist nicht kritisch, sie liegt jedoch bevorzugt im Bereich zwischen etwa —20° und Raumtemperatur.

Verbindungen der Formel III mit Y in der Bedeutung von Halogen lassen sich weiterhin durch Umsetzung von Thioharnstoff mit Oximen der Formel

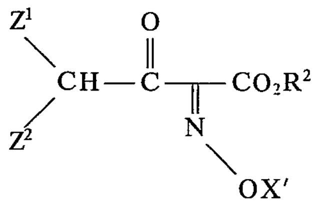

in der $Z^1$ und $Z^2$ gleich oder verschieden sein können und für Brom, Chlor oder Fluor stehen, und X′ sowie $R^2$ die obengenannten Bedeutungen besitzen, durch gegebenenfalls anschließende Einführung von $R^1$ in der Bedeutung einer wie oben definierten, aus der Peptidchemie bekannten Aminoschutzgruppe und/oder durch Verseifung des so erhaltenen Esters der Formel V zur Carbonsäure der allgemeinen Formel III darstellen.

Zweckmäßigerweise erfolgt die Umsetzung mit einer stöchiometrischen Menge Thioharnstoff in einem wasserhaltigen Lösungsmittel, wie z. B. Äthanol oder Aceton. Die Reaktion sollte bei Raumtemperatur durchgeführt werden und maximal etwa 2 bis 3 Stunden dauern.

Verbindungen der Formel III mit Y in der Bedeutung von Methyl lassen sich beispielsweise durch Umsetzung vonThioharnstoff mit Verbindungen der Formel

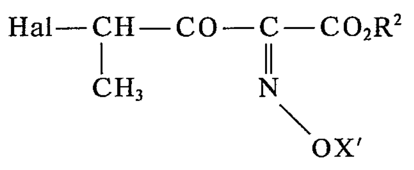

in der Hal Chlor oder Brom bedeutet und X′ sowie $R^2$ die obengenannten Bedeutungen haben, durch gegebenenfalls anschließende Einführung von $R^1$ in der Bedeutung einer wie oben definierten, aus der

Peptidchemie bekannten Aminoschutzgruppe und/oder durch Verseifung des Esters zu Carbonsäuren der Formel III darstellen.

Zweckmäßigerweise setzt man mit einer stöchiometrischen Menge Thioharnstoff in einem wasserhaltigen Lösungsmittel, wie Aceton oder Äthanol, bei Raumtemperatur für maximal etwa 2 bis 3 Stunden um.

Die Ausgangsverbindungen der Formel V sind literaturbekannt oder nach literaturbekannten Verfahren herstellbar.

Steht in den allgemeinen Formeln III und V der Rest $R^1$ für eine leicht entfernbare, aus der Peptidchemie als Aminoschutzgruppe bekannte Gruppe, so kann ihre Einführung in die Aminogruppe auf die aus der Peptidchemie für Aminoschutzgruppen bekannte Weise erfolgen. Stellt $R^1$ beispielsweise die Tritylgruppe dar, so kann ihre Einführung mit Triphenylchlormethan erfolgen, wobei die Reaktion zweckmäßigerweise in einem organischen Lösungsmittel, wie beispielsweise halogenierten Kohlenwasserstoffen in Gegenwart von Basen, wie vorzugsweise Triäthylamin, durchgeführt wird.

Steht in den Formeln III und V X' für eine leicht abspaltbare Gruppe so kann ihre Einführung auf eine Weise geschehen, die dem Fachmann für den Schutz von Hydroxylgruppen geläufig ist.

Nicht nur bei der Darstellung der Ausgangsmaterialien III bzw. V, die eine Gruppe

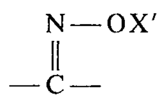

oder

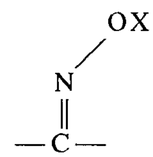

in syn-Position enthalten, sondern auch bei der Darstellung aller Zwischenverbindungen und bei ihrer weiteren Umsetzung zu IV und I ist es zweckmäßig, möglichst milde Reaktionsbedingungen, wie sie dem Fachmann für Reaktionen mit syn-Verbindungen aus der Literatur bekannt sind, wie z. B. keine erhöhte Temperatur, keine verlängerten Reaktionszeiten, keine wesentlichen Überschüsse an sauren Reaktionskomponenten usw. anzuwenden, um ein eventuell mögliches Umklappen der Oxim-Gruppe in die anti-Form zu vermeiden.

Die erfindungsgemäß erhaltenen Verbindungen der Formel I zeigen bemerkenswert gute antibakterielle Wirksamkeiten sowohl gegen grampositive als auch gramnegative bakterielle Keime, die erheblich über denjenigen der Verbindungen liegen, die als nächster Stand der Technik (FR-A-2 294 690) anzusehen sind.

Auch gegen penicillinase- und cephalosporinasebildende Bakterien sind die neuen Verbindungen unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der Formel I können oral, intramuskulär oder intravenös verabfolgt werden.

Arzneipräparate, die eine oder mehrere Verbindungen der allgemeinen Formel I als Wirkstoff enthalten, können hergestellt werden, indem man die Verbindungen der allgemeinen Formel I mit einem oder mehreren pharmakologisch verträglichen Trägerstoffen oder Verdünnungsmitteln, wie z. B. Füllstoffen, Emulgatoren, Gleitstoffen, Geschmacks-korrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Lösung oder Suspension. Als Träger- oder Verdünnungsmittel seien beispielsweise erwähnt, Traganth, Milchzucker, Talkum, Agar-Agar, Polyglykole, Äthanol und Wasser. Für die parenterale Applikation kommen vorzugsweise Suspensionen oder Lösungen in Wasser in Betracht. Es ist auch möglich, die Wirkstoffe als solche ohne Träger- oder Verdünnungsmittel in geeigneter Form beispielsweise in Kapseln zu applizieren.

Geeignete Dosen der Verbindungen der allgemeinen Formel I liegen bei etwa 0,4 bis 20 g/Tag, vorzugsweise bei 0,5 bis 4 g/Tag für einen Erwachsenen von etwa 60 kg Körpergewicht. Es können Einzel- oder im allgemeinen Mehrfachdosen verabfolgt werden, wobei die Einzeldosis den Wirkstoff in einer Menge von etwa 50 bis 1000 mg, vorzugsweise 100 bis 500 mg enthalten kann.

Erfindungsgemäß lassen sich außer den in den Ausführungsbeispielen genannten Verbindungen beispielsweise die folgenden Verbindungen darstellen:

7-[α-syn-Methoximino-α-(2-amino-5-methyl-thiazol-4-yl)-acetamido]-cephalosporansäure-1-S-oxid

7-[α-syn-Methoximino-α-(2-amino-5-brom-thiazol-4-yl)-acetamido]cephalosporansäure-1-S-oxid
7-[α-syn-Äthoximino-α-(2-amino-5-methyl-thiazol-4-yl)-acetamido]-cephalosporansäure, sowie dessen 1-S-oxid
7-[α-syn-Äthoximino-α-(2-amino-5-brom-thiazol-4-yl)-acet-amido]-cephalosporansäure, sowie dessen 1-S-oxid
7-[α-syn-n-Propoximino-α-(2-amino-5-methyl-thiazol-4-yl)-acetamido]-cephalosporansäure, sowie dessen 1-S-oxid
7-[α-syn-n-Propoximino-α-(2-amino-5-brom-thiazol-4-yl)-acetamido]-cephalosporansäure, sowie dessen 1-S-oxid
7-[α-syn-n-Propoximino-α-(2-amino-5-chloro-thiazol-4-yl)-acetamido]-cephalosporansäure, sowie dessen 1-S-oxid
7-[α-syn-iso-Propoximino-α-(2-amino-5-methyl-thiazol-4-yl)-acetamido]-cephalosporansäure, sowie dessen 1-S-oxid
7-[α-syn-iso-Propoximino-α-(2-amino-5-bromo-thiazol-4-yl)-acetamido]-cephalosporansäure, sowie dessen 1-S-oxid
7-[α-syn-iso-Propoximino-α-(2-amino-5-chloro-thiazol-4-yl)-acetamido]-cephalosporansäure, sowie dessen 1-S-oxid
7-[α-syn-n-Butoximino-α-(2-amino-5-methyl-thiazol-4-yl)-acetamido]-cephalosporansäure, sowie dessen 1-S-oxid
7-[α-syn-n-Butoximino-α-(2-amino-5-chloro-thiazol-4-yl)-acetamido]-cephalosporansäure, sowie dessen 1-S-oxid
7-[α-syn-Carboxymethoximino-α-(2-amino-5-methyl-thiazol-4-yl)-acetamido]-cephalosporansäure, sowie dessen 1-S-oxid
7-[α-syn-Carboxymethoximino-α-(2-amino-5-bromo-thiazol-4-yl)-acetamido]-cephalosporansäure, sowie dessen 1-S-oxid
7-[α-syn-Äthoxycarbonyl-methoximino-α-(2-amino-5-methyl-thiazol-4-yl)-acetamido]-cephalosporansäure, sowie dessen 1-S-oxid
7-[α-syn-Äthoxycarbonyl-methoximino-α-(2-amino-5-bromo-thiazol-4-yl)-acetamido]-cephalosporansäure, sowie dessen 1-S-oxid
7-[α-syn-Äthoxycarbonyl-methoximino-α-(2-amino-5-chloro-thiazol-4-yl)-acetamido]-cephalosporansäure, sowie dessen 1-S-oxid
7-[α-syn-Carboxymethoximino-α-(2-amino-5-chloro-thiazol-4-yl)-acetamido]-cephalosporansäure, sowie dessen 1-S-oxid
7-[α-syn-Cyanomethoximino-α-(2-amino-5-methyl-thiazol-4-yl)-acetamido]-cephalosporansäure, sowie dessen 1-S-oxid
7-[α-syn-Cyanomethoximino-α-(2-amino-5-bromo-thiazol-4-yl)-acetamido]-cephalosporansäure, sowie dessen 1-S-oxid
7-[α-syn-Cyanomethoximino-α-(2-amino-5-chloro-thiazol-4-yl)-acetamido]-cephalosporansäure, sowie dessen 1-S-oxid
7-[α-syn-Aminocarbonylmethoximino-α-(2-amino-5-methyl-thiazol-4-yl)-acetamido]-cephalosporansäure, sowie dessen 1-S-oxid
7-[α-syn-Aminocarbonyl-methoximino-α-(2-amino-5-brom-thiazol-4-yl)-acetamido]-cephalosporansäure, sowie dessen 1-S-oxid
7-[α-syn-Aminocarbonylmethoximino-α-(2-amino-5-chloro-thiazol-4-yl)-acetamido]-cephalosporansäure, sowie dessen 1-S-oxid
7-[α-syn-Hydroximino-α-(2-amino-5-methyl-thiazol-4-yl)-acetamido]-cephalosporansäure, sowie dessen 1-S-oxid
7-[α-syn-Hydroximino-α-(2-amino-5-bromo-thiazol-4-yl)-acetamido]-cephalosporansäure, sowie dessen 1-S-oxid
7-[α-syn-Hydroximino-α-(2-amino-5-chloro-thiazol-4-yl)-acetamido]-cephalosporansäure, sowie dessen 1-S-oxid
7-[α-syn-Hydroximino-α-(2-amino-5-fluoro-thiazol-4-yl)-acetamido]-cephalosporansäure, sowie dessen 1-S-oxid
7-[α-syn-Methoximino-α-(2-amino-5-fluoro-thiazol-4-yl)-acetamido]-cephalosporansäure, sowie dessen 1-S-oxid
7-[α-syn-Äthoximino-α-(2-amino-5-fluoro-thiazol-4-yl)-acetamido]-cephalosporansäure, sowie dessen 1-S-oxid
7-[α-syn-n-Propoximino-α-(2-amino-5-fluoro-thiazol-4-yl)-acetamido]-cephalosporansäure, sowie dessen 1-S-oxid
7-[α-syn-iso-Propoximino-α-(2-amino-5-fluoro-thiazol-4-yl)-acetamido]-cephalosporansäure, sowie dessen 1-S-oxid
7-[α-syn-n-Butoximino-α-(2-amino-5-fluoro-thiazol-4-yl)-acetamido]-cephalosporansäure, sowie dessen 1-S-oxid
7-[α-syn-Carboxymethoximino-α-(2-amino-5-fluoro-thiazol-4-yl)-acetamido]-cephalosporansäure, sowie dessen 1-S-oxid

7-[α-syn-Äthoxycarbonylmethoximino-α-(2-amino-5-fluoro-thiazol-4-yl)-acetamido]-cephalosporansäure, sowie dessen 1-S-oxid
7-[α-syn-Cyanomethoximino-α-(2-amino-5-fluoro-thiazol-4-yl)-acetamido]-cephalosporansäure, sowie dessen 1-S-oxid
7-[α-syn-Aminocarbonylmethoximino-α-(2-amino-5-fluoro-thiazol-4-yl)-acetamido]-cephalosporansäure, sowie dessen 1-S-oxid

Die Beispiele erläutern die Erfindung, ohne sie jedoch einzuschränken.

Die in den Beispielen angegebenen $R_F$-Werte wurden durch Dünnschichtchromatographie auf Kieselgel-Fertigplatten 60 F 254 der Firma Merck, Darmstadt, ermittelt.

## Beispiel 1

### 7-[α-syn-Methoximino-α-(2-amino-5-methyl-thiazol-4-yl)-acetamido]-cephalosporansäure

#### Stufe 1

43 g Propionylessigsäureäthylester (0,3 Mol) in 45 ml Eisessig wurden bei 20—25° unter Rühren und Kühlen langsam mit einer Lösung aus 22,8 g Natriumnitril (0,3 Mol) in 45 ml $H_2O$ versetzt. Es wurde 1 h bei 10° gerührt, mit 90 ml Wasser versetzt, 1 h bei Raumtemperatur gerührt, und viermal mit je 75 ml Äther extrahiert. Die vereinigten Ätherextrakte wurden mit $NaHCO_3$-Lösung neutral geschüttelt, über Natriumsulfat getrocknet und zur Trockne eingedampft.

Man erhielt so 55 g α-Oximino-propionylessigsäureäthylester als gelbes Öl, das bei tiefer Temperatur kristallisiert;
$n_D^{20}$ des Öls: 1,4413, $R_F$: 0,3 ($CHCl_3$/Aceton = 20/1). Das Öl wurde ohne weitere Reinigung eingesetzt.

#### Stufe 2

Das in Stufe 1 gewonnene Öl wurde in 250 ml Aceton gelöst, mit 60 g $K_2CO_3$ versetzt und innerhalb von 30 min mit 38,5 g Dimethylsulfat (0,3 Mol) versetzt. Nach zweistündigem Rühren bei Raumtemperatur wurde auf 200 g Eis gegossen, mit 500 ml $H_2O$ verrührt, einmal mit 150 ml Äther und dreimal mit $CH_2Cl_2$ extrahiert. Die vereinigten organischen Extrakte wurden nach Trocknen über $Na_2SO_4$ vom Lösungsmittel befreit und lieferten 45 g α-Methoximino-propionylessigsäure-äthylester als Öl.

$R_F$: 0,63 ($CHCl_3$/Aceton = 20/1)

NMR (DMSO-$d_6$): $\delta$ = 0,9—1,4 ppm (2 × tr., 6H, $OCH_2CH_3$ und $COCH_2CH_3$)
$\delta$ = 2,85 ppm (q, 2H, $COCH_2CH_3$)
$\delta$ = 4,05 ppm, $\delta$ = 4,10 ppm } (2 × s, 1 : 4; 3H, $OCH_3$)
$\delta$ = 4,30 ppm (q, 2H, $OCH_2CH_3$)

Das Produkt wurde ohne weitere Reinigung eingesetzt:

#### Stufe 3

45 g des in Stufe 2 gewonnenen Öls wurden in 300 ml $CH_2Cl_2$ gelöst, auf 20° gekühlt und mit einer Lösung aus 39 g $Br_2$ (0,24 Mol) in 50 ml $CH_2Cl_2$ tropfenweise versetzt. Anschließend wurde noch 1 1/2 h bei Raumtemperatur gerührt, mit zweimal je 300 ml $H_2O$ ausgeschüttelt, über $Na_2SO_4$ getrocknet und im Vakuum vom Lösungsmittel befreit. Man erhält so 60 g α-Methoximino-γ-brom-propionyl-essigsäure-äthylester als hellbraunes Öl.

$R_F$ = 0,38 ($CHCl_3$)

Das Produkt wurde ohne weitere Reinigung eingesetzt.

#### Stufe 4

13,3 g des in Stufe 3 gewonnenen Öls wurden in 13 ml Äthanol gelöst zu einer Lösung aus 3,8 g (0,05 Mol) Thioharnstoff in 13 ml Äthanol und 27 ml $H_2O$ bei 16—18° gegeben. Nach Zugabe weiterer 13 ml

Äthanol wurde 1 Stunde bei Raumtemperatur gerührt.

Unter pH-Kontrolle wurde mit gesättigter $KHCO_3$-Lösung auf pH 5 eingestellt. Durch Abfiltrieren wurden 4,6 g (56%) α-syn-Methoximino-α-(2-amino-5-methyl-thiazol-4-yl)-essigsäureäthylester gewonnen.

F: 135—136°

NMR (DMSO-$d_6$): $\delta$ = 1,2 ppm (t, 3H, $OCH_2-CH_3$)
$\delta$ = 4,25 ppm (q, 2H, $OCH_2-CH_3$)
$\delta$ = 142 Hz (s, 3H, $CH_3$, syn)
$\delta$ = 232 Hz (s, 3H, $OCH_3$, syn)

Stufe 5

12,2 (0,05 Mol) des in Stufe 4 gewonnenen Esters wurden in 23,7 ml DMF gelöst, mit 47,5 ml $CH_2Cl_2$ versetzt und auf −10° gekühlt. Nach Zugabe von 7,35 ml Triäthylamin wurde auf −35° gekühlt und unter Rühren in Portionen mit insgesamt 16,5 g Tritylchlorid versetzt. Die Lösung wurde $2^1/_2$ Stunden ohne Kühlung gerührt, mit 100 ml $CH_2Cl_2$ verdünnt und nacheinander mit zweimal 50 ml 1 n HCl und dreimal 100 ml $H_2O$ gewaschen. Die organische Phase wurde über $Na_2SO_4$ getrocknet und im Vakuum vom Lösungsmittel befreit.

Das so gewonnene Öl wurde ohne weitere Reinigung eingesetzt.

Stufe 6

Das in Stufe 5 gewonnene Öl wurde in 240 ml Äthanol gelöst und mit 10 ml 10 n NaOH versetzt. Nach fünfstündigem Rühren bei Raumtemperatur wurde abfiltriert.

Man erhielt so 10,3 g des Natriumsalzes von α-syn-Methoximino-α-(2-tritylamino-5-methyl-thiazol-4-yl)-essigsäure, das ohne weitere Reinigung eingesetzt wurde.

Stufe 7

9,6 g (20 mMol) des in Stufe 6 gewonnenen Natriumsalzes wurden in 80 ml $CH_2Cl_2$ und 10 ml Äther Eiskühlung mit 23 ml 2 n HCl versetzt. Die $CH_2Cl_2$ wurde abgetrennt und die wäßrige Phase nochmals mit 10 ml $CH_2Cl_2$ extrahiert. Nach Trocknen der vereinigten $CH_2Cl_2$-Phasen über $Na_2SO_4$ und Entfernen des Lösungsmittels im Vakuum verblieb ein Schaum:
α-syn-Methoximino-α-(2-tritylamino-5-methyl-thiazol-4-yl)-essigsäure, die ohne weitere Reinigung eingesetzt wurde.

Stufe 8

Das in Stufe 7 gewonnene Produkt wurde in 30 ml $CH_2Cl_2$ gelöst, unter $N_2$ im Eisbad gekühlt und mit 2,35 g Dicyclohexylcarbodiimid versetzt. Es wurde $^1/_2$ h bei 0° und 1 h bei Raumtemperatur gerührt, vom gebildeten Dicyclohexylharnstoff abfiltriert, auf −20° gekühlt und mit einer Lösung von 2,72 g 7-Aminocephalosporansäure und 3,3 ml Triäthylamin in 40 ml $CH_2Cl_2$ versetzt. Es wurde $2^1/_2$ h bei Raumtemperatur gerührt und anschließend mit 1 n HCl auf pH 2,75 gestellt.

Die $CH_2Cl_2$-Phase wurde zweimal mit 70 ml $H_2O$ gewaschen, getrocknet über $Na_2SO_4$ und im Vakuum zur Trockne eingedampft. Der Rückstand wurde in einem Gemisch aus 50 ml Dioxan/50 ml Äther aufgenommen, mit 2,1 ml (20 mMol) Diäthylamin versetzt, und mit Äther bis zur beginnenden Trübung versetzt. Nach 48 Stunden bei −5° konnten durch Filtration 2,6 g Diäthylammoniumsalz der α-syn-Methoximino-α-(2-tritylamino-5-methyl-thiazol-4-yl)-essigsäure gesammelt werden.

Durch Eindampfen der Mutterlauge ließen sich 7,0 g eines Gemisches der Diäthylammoniumsalze von α-syn-Methoximino-(2-tritylamino-5-methyl-thiazol-4-yl)-essigsäure und 7-[α-syn-Methoximino-α-(2-tritylamino-5-methyl-thiazol-4-yl)-acetamido]-cephalosporansäure gewinnen.

Stufe 9

7,0 g des in Stufe 8 gewonnenen Diäthylammoniumsalzgemisches wurden in einem Gemisch aus 30 ml 98% Ameisensäure und 20 ml $H_2O$ gelöst und $2^1/_2$ Stunden bei Raumtemperatur gerührt. Es wurde vom gebildeten Triphenylcarbinol abfiltriert, auf 250 ml mit $H_2O$ verdünnt, erneut abfiltriert und am Rotationsverdampfer auf ca. 30 ml eingeengt.

Mit gesättigter $NaHCO_3$-Lösung wurde auf pH 8,0 gestellt, mit Essigester extrahiert, mit 50 ml Essigester überschichtet und mit 2 n HCl auf pH 2,0 gebracht. Die wäßrige Lösung wurde fünfmal mit Essigester extrahiert, die vereinigten Essigesterextrakte wurden über $Na_2SO_4$ getrocknet und am Rotationsverdampfer eingeengt. Das entstandene Öl wird mit 20 ml Äther versetzt und 1 Stunde gerührt, wobei es sich verfestigt.

Der Niederschlag wird abgesaugt.

Es wurden so 1,5 g 7-[α-syn-Methoximino-α-(2-aminothiazol-4-yl)-acetamido]-cephalosporansäure mit dem $R_F$-Wert 0,53 (Essigester : iso-Propanol : Wasser = 6 : 4 : 3) erhalten.

F: 125—135° (Z)

NMR (DMSO-$d_6$): δ = 2,0 ppm (s, 3H, $OCOCH_3$)
δ = 2,3 ppm (s, 3H, Thiazol—$CH_3$)
δ = 3,8 ppm [231 Hz] (s, 3H, N—$OCH_3$, syn)
δ = 4,7 ppm (AB, 2H, 3'—$CH_2$)
δ = 5,1 ppm (d, 1H, 6-H)
δ = 5,6 ppm (q, 1H, 7-H)

## Beispiel 2

### 7-[α-syn-Methoximino-α-(2-amino-5-bromo-thiazol-4-yl)-acetamido]-cephalosporansäure

### Stufe 1

9,15 g α-syn-Methoximino-α-(2-amino-thiazol-4-yl)-essigsäureäthylester (40 mMol) gelöst in 50 ml Eisessig wurden bei 15° tropfenweise mit 6,39 g Brom in 20 ml Eisessig versetzt. Nach beendeter Zugabe wurde noch 15 min nachgerührt und auf 150 g Eis gegeben. Der Niederschlag wurde abfiltriert, mit Wasser gewaschen:

Es konnten so 10,9 g α-syn-Methoximono-α-(2-amino-5-bromo-thiazol-4-yl)-essigsäure-äthylester mit F = 149—151° gewonnen werden. Umkristallisieren aus Äthanol:

F: 157—158°

NMR (DMSO-$d_6$): δ = 1,27 ppm (t, 3H, $OCH_2CH_3$)
δ = 3,93 ppm [236 Hz] (s, 3H, $NOCH_3$)
δ = 4,3 ppm (q, 2H, $OCH_2CH_3$)
δ = 7,4 ppm (s, breit, 2H, $NH_2$)

### Stufe 2

3 g des in Stufe 1 erhaltenen Esters wurden in 8 ml Methanol gelöst, und bei 50°C mit 20 ml 80% Hydrazinhydrat versetzt. Es wurde 4 Stunden bei Raumtemperatur gerührt, auf 0°C abgekühlt und vom Niederschlag abfiltriert. Man erhielt so 2,5 g α-syn-Methoximino-α-(2-amino-5-bromo-thiazol-4-yl)-acethydrazin mit F = 200° (Z).

Die Kristalle wurden, da sie sich sehr rasch zersetzten sofort weiter verarbeitet.

### Stufe 3

1,5 g des in Stufe 2 dargestellten Hydrazids wurden in 25 ml DMF gelöst, auf −20°C gekühlt, mit 3,3 ml einer 4,51 N HCl in Dioxan und anschließend langsam mit 0,6 ml tert.-Butylnitrit in 4 ml Dioxan versetzt.

Die grellgelbe Lösung wurde 1/2 Stunde bei −20° gerührt, und mit 1,5 g Triäthylamin in 10 ml Dioxan versetzt. Anschließend wurde eine Lösung aus 1,36 g 7-Aminocephalosporansäure in 10 ml DMF und 1,0 g $NEt_3$ zugetropft. Innerhalb der folgenden Stunde wurden noch weitere 0,5 g Triäthylamin in 6 ml Dioxan in 3 Portionen zugegeben. Es wurde in 100 ml $H_2O$ gegeben, dreimal mit Essigester extrahiert, auf pH 4,0 gestellt, vom Unlöslichen abfiltriert, und weiter mit 2 N HCl auf pH 1,5 angesäuert.

Es wurde fünfmal mit Essigester extrahiert, die vereinigten Essigesterextrakte wurden nach Trocknen über $Na_2SO_4$ eingeengt. Es entstand ein Öl, das nach Anreiben mit Äther fest wurde. Es konnten so 650 mg 7-[α-syn-Methoximino-α-(2-amino-5-bromo-thiazol-4-yl)-acetamido]-cephalosporansäure gewonnen werden.

F: 135—145° (Z)

IR (KBr): 1770 $cm^{-1}$ (β-Lactam)
1720 $cm^{-1}$ (Acetat)

NMR (DMSO-$d_6$): = 2,0 ppm (s, 3H, $OCOCH_3$)
= 3,9 ppm [232 Hz] (s, 3H, $NOCH_3$)
= 9,5 ppm (d, 1H, —NH—CO—)

Beispiel 3

7-[α-syn-n-Butoximino-α-(2-amino-5-bromo-thiazol-4-yl)-acetatamido]-cephalosporansäure

Stufe 1

11 g α-syn-n-Butoximino-α-(2-amino-thiazol-4-yl)-essigsäureäthylester lieferten mit 6,4 g Brom analog der in Beispiel 2 Stufe 1 beschriebenen Weise 11,7 g α-syn-n-Butoximino-α-(2-amino-5-bromo-thiazol-4-yl)-essigsäureäthylester mit F = 130—140° (Äthanol).

NMR (DMSO-$d_6$): = 0,7—1,9 ppm (m, 10H, $-CH_2-CH_2-CH_3$ und $-OCH_2CH_3$)
= 4,15 ppm [248 Hz] (t, 2H, $-OCH_2-CH_2-$)
= 4,3 ppm (q, 2H, $-O-CH_2-CH_3$)

Stufe 2

3,2 g des in Stufe 1 erhaltenen Esters lieferten analog der in Beispiel 2, Stufe 2 beschriebenen Weise 2,8 g α-syn-n-Butoximino-α-(2-amino-5-bromo-thiazol-4-yl)-acethydrazid.

F = ~190° (Z)

Das Produkt wurde wegen seiner Zersetzlichkeit sofort weiter verarbeitet.

Stufe 3

1,68 g des in Stufe 2 gewonnenen Hydrazides lieferten analog der in Beispiel 2, Stufe 3 beschriebenen Weise 520 mg 7-[α-syn-n-Butoximino-α-(2-amino-5-bromo-thiazol-4-yl)-acetamido]-cephalosporansäure.

F: 140—150° (Z)

IR (KBr): 1770 $cm^{-1}$ (β-Lactam)
1725 $cm^{-1}$ ($OCOCH_3$)

NMR (DMSO-$d_6$): δ = 0,7—1,9 ppm (m, 7H, $CH_2-CH_2-CH_3$)
δ = 2,07 ppm (s, 3H, $OCOCH_3$)
δ = 4,15 ppm [244 Hz] (t, 2H, $-O-CH_2-CH_2$)
δ = 9,4 ppm (d, 1H, NHCO)

Beispiel 4

7-[α-syn-Methoximino-α-(2-amino-5-chloro-thiazol-4-yl)-acetamido]-cephalosporansäure

Stufe 1

50 g α-syn-Methoximino-α-(2-amino-thiazol-4-yl)-essigsäure wurden in einem Gemisch aus 300 ml $CHCl_3$ und 150 ml Eisessig aufgeschlämmt und bei 0—10° unter Rühren mit 17,5 g $Cl_2$ in 200 ml Eisessig versetzt. Es wurde 1/2 Stunde bei 0° gerührt, und vom Niederschlag abfiltriert. Es wurde nochmals in $CHCl_3$ aufgeschlämmt und erneut abgesaugt.

Der Filterrückstand wurde in 190 ml Tetrahydrofuran 15 min refluxiert und 2 Stunden bei Raumtemperatur gerührt. Man erhielt so 30,3 g α-syn-Methoximino-α-(2-amino-5-chloro-thiazol-4-yl)-essigsäure × 1 HCl × 1 $H_2O$ × 1 Tetrahydrofuran.

F: 150—153°.

Stufe 2

29 g der in Stufe 1 gewonnenen Säure wurden in 100 ml Methanol gelöst und mit 4,85 g Natrium-Methylat versetzt. Nach fünfzehnminütigem Rühren bei RT wurde zur Trockne im Vakuum eingedampft, und der Rückstand dreimal mit je 75 ml trockenem THF in der Siedehitze extrahiert. Die filtrierten THF-Mutterlaugen wurden eingedampft, und der Rückstand aus sehr wenig Methanol umkristallisiert. Man erhielt so 13,5 g α-syn-Methoximino-α-(2-amino-5-chlorothiazol-4-yl)-essigsäure × 1 Methanol.

F: 129—130° (Z)

NMR ($DMSO-d_6$): $\delta$ = 3,1 ppm (s, 3H, $CH_3OH$)
$\delta$ = 3,9 ppm [234 Hz] (s, 3H, N—$OCH_3$)

Stufe 3

8,0 g der in Stufe 2 gewonnenen Carbonsäure wurden in 100 ml Dimethylacetamid gelöst, mit 100 ml Tetrachlorkohlenstoff versetzt und am Rotationsverdampfer von Tetrachlorkohlenstoff befreit, um das in der Stufe 2 enthaltene Methanol zu entfernen. Anschließend wurde auf −20° gekühlt, und mit 3,4 g Chloracetylchlorid, gelöst in 10 ml Dimethylacetamid, versetzt. Die Temperatur wurde 1/4 h bei −20° C, 1/2 h bei 0° C und anschließend 1/4 h bei +10° gehalten.
Es wurde auf Eis gegeben und mit Essigester dreimal extrahiert. Nach dem Trocknen über $Na_2SO_4$ und Abdampfen des Lösungsmittels verblieben 8,2 g α-syn-Methoximino-α-(2-chloracetylamino-5-chlor-thiazol-4-yl)-essigsäure als schwach gelbes Öl.

$R_F$: 0,54 (Essigester : iso-Propanol : $H_2O$ = 6 : 4 : 3).

NMR ($DMSO-d_6$): $\delta$ = 3,9 ppm [232 Hz] (s, 3H, $NOCH_3$)
$\delta$ = 4,4 ppm (s, 2H, $COCH_2Cl$)

Stufe 4

3,1 g der in Stufe 3 dargestellten Carbonsäure wurden in 15 ml $CH_2Cl_2$ gelöst und mit 1,45 ml Triäthylamin versetzt. Es wurde auf 0° abgekühlt und mit 0,5 ml Thionylchlorid, gelöst in 5 ml Methylenchlorid tropfenweise versetzt. Es wurde 1/4 Stunde bei 0° gerührt und nach Zugabe von weiteren 1,3 ml Triäthylamin mit einer Lösung von 2,5 g 7-Aminocephalosporansäure in 20 ml Methylenchlorid und 2,8 ml Triäthylamin versetzt. Es wurde 1 Stunde nachgerührt, im Vakuum vom Lösungsmittel befreit und in 40 ml $H_2O$ aufgenommen. Der pH wurde auf 7,0 eingestellt, die Lösung zweimal mit 20 ml Essigester extrahiert, auf pH 4,0 gestellt, von der ausgefallenen 7-Aminocephalosporansäure befreit und weiter auf pH 2,0 angesäuert. Durch Filtration konnten 1,5 g 7-[α-syn-Methoximino-α-(2-chloracetyl-amino-5-chloro-thiazol-4-yl)-acetamido]-cephalosporansäure gesammelt werden.

$R_F$: 0,49 (Essigester : iso-Propanol : $H_2O$ = 6 : 4 : 3)

NMR ($DMSO-d_6$): $\delta$ = 2,05 ppm (s, 3H, $OCOCH_3$)
$\delta$ = 3,9 ppm [234 Hz] (s, 3H, $NOCH_3$)
$\delta$ = 4,4 ppm (s, 2H, $COCH_2Cl$)
$\delta$ = 9,7 ppm (d, 1H, —NH—CO—)

Stufe 5

2,9 g des in Stufe 4 dargestellten Produktes wurden in 50 ml eines 1 : 1 Gemisches aus Äthanol und Tetrahydrofuran mit 400 mg Thioharnstoff versetzt und 15 Stunden bei Raumtemperatur gerührt. Es wird im Vakuum zur Trockne eingeengt und in 20 ml Wasser aufgenommen.

Es wird abfiltriert, nochmals in einem Gemisch Wasser/Äthanol 1 : 1, 10 ml aufgerührt und abfiltriert. Man erhält so 0,92 g 7-[α-syn-Methoximino-α-(2-amino-5-chloro-thiazol-4-yl)-acetamino]-cephalosporansäure.

F = 135—145° (Z)

IR (KBr): 1770 $cm^{-1}$ (β-Lactam)
1720 $cm^{-1}$ (Acetatbande)

NMR (DMSO-$d_6$): δ = 2,0 ppm (s, 3H, $OCOCH_3$)
δ = 3,85 ppm [231 Hz] (s, 3H, = $NOCH_3$)
δ = 9,5 ppm (d, 1H, NHCO)

## Beispiel 5

7-[α-syn-Äthoximino-α-(2-amino-5-chloro-thiazol-4-yl)-acetamido]-cephalosporansäure

### Stufe 1

54 g α-syn-Äthoximino-α-(2-amino-thiazol-4-yl)-essigsäure lieferten analog der in Beispiel 4, Stufe 1 beschriebenen Weise 32,1 g α-syn-Äthoximino-α-(2-amino-5-chloro-thiazol-4-yl)-essigsäure × 1 HCl × 1 Tetrahydrofuran:

F: 106—108° (Z)

### Stufe 2

30,7 g der in Stufe 1 gewonnenen Säure lieferten analog der in Beispiel 4, Stufe 2 beschriebenen Weise 18,4 g α-syn-Äthoximino-α-(2-amino-5-chloro-thiazol-4-yl)-essigsäure × 1 Methanol.

NMR (DMSO-$d_6$): δ = 3,15 ppm (s, 3H, $CH_3OH$)
δ = 1,2 ppm (t, 3H, $OCH_2CH_3$)
δ = 4,15 ppm [250 Hz] (q, 2H, $OCH_2CH_3$)

### Stufe 3

8,4 g der in Stufe 2 beschriebenen Carbonsäure lieferten analog der in Beispiel 4, Stufe 3 beschriebenen Weise 7,4 g α-syn-Äthoximino-α-(2-chloracetyl-amino-5-chloro-thiazol-4-yl)-essigsäure als schwachgelbes Öl.

$R_F$: 0,56 (Essigester : iso-Propanol : $H_2O$ = 6 : 4 : 3)

NMR (DMSO-$d_6$): δ = 1,25 ppm (t, 3H, $OCH_2CH_3$)
δ = 4,15 ppm [248 Hz] (q, 2H, $OCH_2CH_3$)
δ = 4,35 ppm (s, 2H, $COCH_2Cl$)

### Stufe 4

3,25 g der in Stufe 3 hergestellten Carbonsäure lieferten analog der in Beispiel 4, Stufe 4 beschriebenen Weise 1,9 g 7-[α-syn-Äthoximino-α-(2-chloracetylamino-5-chloro-thiazol-4-yl)-acetamido]-cephalosporansäure.

$R_F$ = 0,51 (Essigester : iso-Propanol : $H_2O$ = 6 : 4 : 3)

NMR (DMSO-$d_6$): δ = 1,2 ppm (t, 3H, $OCH_2CH_3$)
δ = 4,1 ppm [250 Hz] (q, 2H, $OCH_2CH_3$)
δ = 2,05 ppm (s, 3H, $OCOCH_3$)
δ = 4,4 ppm (s, 2H, $COCH_2Cl$)
δ = 9,85 ppm (d, 1H, NHCO)

Stufe 5

2,8 g des in Stufe 4 hergestellten Produktes lieferten analog der in Beispiel 4, Stufe 5 beschriebenen Weise 860 mg 7-[α-syn-Äthoximino-α-(2-amino-5-chloro-thiazol-4-yl)-acetamido]-cephalosporansäure:

F: 140—150° (Z)

IR (KBr): 1775 $cm^{-1}$ (β-Lactam)
1725 $cm^{-1}$ (O-Acetat)

NMR (DMSO-$d_6$): = 1,25 ppm (t, 3H, $OCH_2CH_3$)
= 2,0 ppm (s, 3H, $OCOCH_3$)
= 4,1 ppm [251 Hz] (q, 2H, $OCH_2CH_3$)
= 9,7 ppm (d, 1H, NHCO)

Beispiel 6

7-[α-syn-Methoximino-α-(2-amino-5-chloro-thiazol-4-yl)-acetamido]-cephalosporansäure-1-S-oxid

980 mg des in Beispiel 4, Stufe 5 beschriebenen Produktes wurden in 8 ml 98% Ameisensäure und 2 ml Methanol gelöst. Es wurde in Eis gekühlt und mit 410 mg 85% m-Chlorperbenzoesäure, gelöst in 3 ml Tetrahydrofuran, unter Eiskühlung versetzt. Nach einstündigem Rühren bei Raumtemperatur wurde die Lösung in 100 ml Äther getropft, 1 Stunde gerührt und abfiltriert. Der Rückstand wurde erneut in 10 ml Äther aufgenommen und 1/4 Stunde gerührt. Durch Filtration konnten 850 mg der Titelverbindung gewonnen werden.

F > 300°

IR (KBr): 1775 $cm^{-1}$ (β-Lactam)
1710 $cm^{-1}$ ($OCOCH_3$)
1030 $cm^{-1}$ (S → O)

NMR (DMSO-$d_6$): = 2,0 ppm (s, 3H, $OCOCH_3$)
= 3,85 ppm [234 Hz] (s, 3H, $NOCH_3$)
= 8,7 ppm (d, 1H, NHCO)

Beispiel 7

7-[α-syn-Äthoximino-α-(2-amino-5-chloro-thiazol-4-yl)-acetamido]-cephalosporansäure-1-S-oxid

1,06 g des in Beispiel 5, Stufe 5 hergestellten Produktes lieferten analog der in Beispiel 6 angegebenen Weise 910 mg der Titelverbindung.

F > 300°

IR (KBr): 1770 $cm^{-1}$ (β-Lactam)
1710 $cm^{-1}$ ($OCOCH_3$)
1025 $cm^{-1}$ (S → O)

NMR (DMSO-$d_6$): = 1,25 ppm (t, 3H, $OCH_2CH_3$)
= 2,05 ppm (s, 3H, $OCOCH_3$)
= 4,1 ppm [250 Hz] (q, 2H, $OCH_2CH_3$)
= 8,65 ppm (d, 1H, CONH)

**Patentansprüche**

1. Verbindungen der allgemeinen Formel I

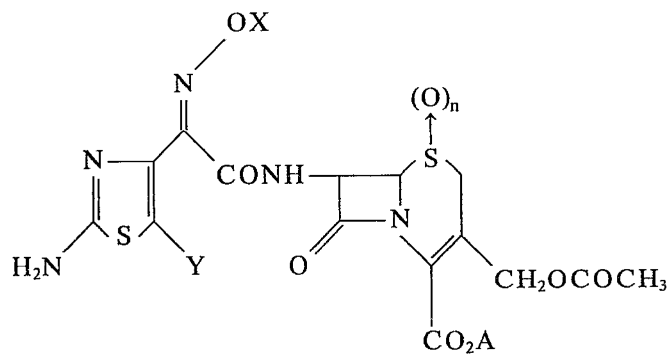

(I)

worin
n = 0 oder 1 und X Wasserstoff, Alkyl mit 1 bis 4 C-Atomen, Carboxymethyl, Alkoxycarbonylmethyl mit 1 bis 4 C-Atomen in der Alkoxygruppe, Aminocarbonylmethyl oder Cyanomethyl bedeuten, wobei die Carboxymethyl-, Alkoxycarbonylmethyl-, Aminocarbonylmethyl- und Cyanomethylgruppe an ihrer Methylengruppe auch noch durch Alkyl mit 1—4 C-Atomen substituiert sein können, Y für Methyl oder Halogen und A für Wasserstoff, einen leicht abspaltbaren Esterrest oder ein physiologisch unbedenkliches Kation steht und die Gruppe =N—OX in der syn-Form vorliegt.

2. Verfahren zur Herstellung von Verbindungen der allgemeinen Formel I gemäß Anspruch 1, dadurch gekennzeichnet, daß man Lactame der allgemeinen Formel II

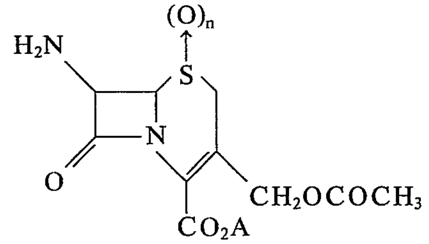

(II)

in der n und A die oben angegebenen Bedeutungen haben, mit einer Carbonsäure der allgemeinen Formel III

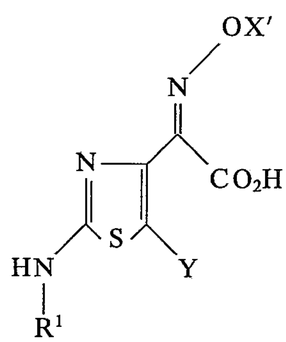

(III)

oder einem aktivierten Derivat derselben, worin Y die vorstehende Bedeutung besitzt,
$R^1$ für Wasserstoff oder für eine aus der Peptidchemie bekannte Aminoschutzgruppe und X' für X, eine leicht abspaltbare Gruppe oder für eine Gruppe der Formel

$$—CH_2CO_2R^3$$

steht, in der $R^3$ die Bedeutung eines unter milden Bedingungen abspaltbaren Restes hat,

zu einer Verbindung der allgemeinen Formel IV

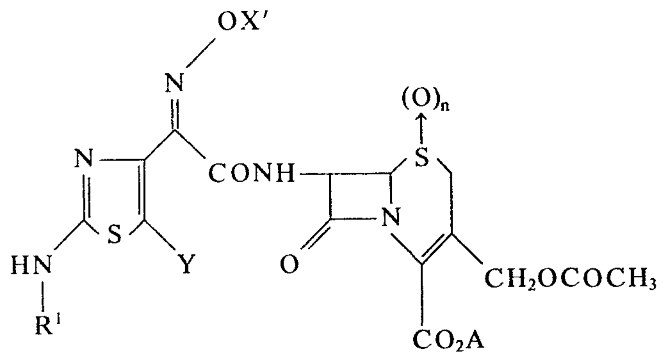

(IV)

in der $R^1$, X', Y, A und n die obengenannten Bedeutungen haben, umsetzt und gegebenenfalls das so erhaltene Produkt am Schwefel des Cephemringes oxidiert und/oder einen Rest $R^1$ in der Bedeutung einer Schutzgruppe abspaltet und/oder den Rest X', soweit er nicht die Bedeutung von X hat, in den Rest X überführt.

3. Pharmazeutische Präparate, gekennzeichnet durch einen Gehalt an Verbindungen der allgemeinen Formel I gemäß Anspruch 1.

4. Verfahren zur Herstellung von pharmazeutischen Präparaten, dadurch gekennzeichnet, daß eine Verbindung der allgemeinen Formel I gemäß Anspruch 1, gegebenenfalls mit pharmazeutisch üblichen Trägerstoffen oder Verdünnungsmitteln, in eine pharmazeutisch geeignete Verabreichungsform gebracht wird.

## Claims

1. Compounds of the general formula I

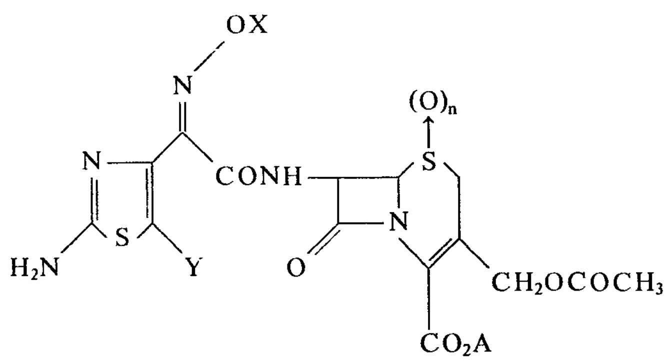

(I)

wherein n denotes 0 or 1 and X represents hydrogen, alkyl with 1 to 4 C-atoms, carboxymethyl, alkoxycarbonylmethyl with 1 to 4 C-atoms in the alkoxy part, aminocarbonylmethyl or cyanomethyl, wherein the carboxymethyl, alkoxycarbonylmethyl, aminocarbonylmethyl and cyanomethyl group may also be substituted in the methylene group by alkyl with 1 to 4 C-atoms, Y represents methyl or halogen and A represents hydrogen, an ester radical which can be easily split off or a physiologically acceptable cation and the group =N—OX is present in the syn-form.

2. Process for the manufacture of compounds of the general formula I, which comprises reacting lactams of the general formula II

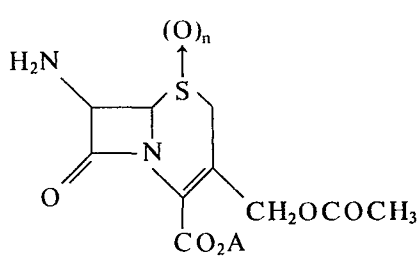

(II)

in which n and A have the meanings indicated above, with a carboxylic acid of the general formula III

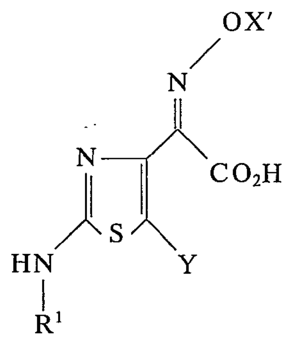

(III)

or an activated derivative thereof, wherein Y has the above meaning, $R^1$ represents hydrogen or represents an aminoprotective group which is known from peptide chemistry and X′ represents X, a group which can be easily split off or represents a group of the formula

$$—CH_2CO_2R^3$$

in which $R^3$ denotes a radical which can be split off under mild conditions, to give a compound of the general formula IV

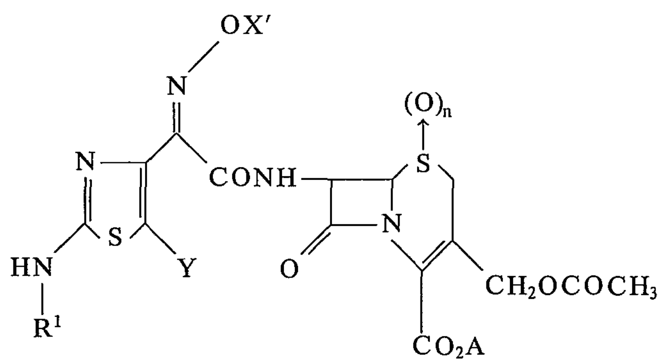

(IV)

in which $R^1$, X′, Y, A and n have the abovementioned meanings, and optionally oxidizing the resulting product on the sulfur of the cephem ring, and/or splitting off a radical $R^1$ in the meaning of a protective group, and/or converting the radical X′, if it does not denote X, into the radical X.

3. Pharmaceutical formulations containing compounds of the general formula I.

4. Process for the manufacture of pharmaceutical formulations, which comprises bringing a compound of the general formula I into a pharmaceutically suitable form for administration, if appropriate with pharmaceutically customary excipients or diluents.

**Revendications**

1. Composés de formule générale I:

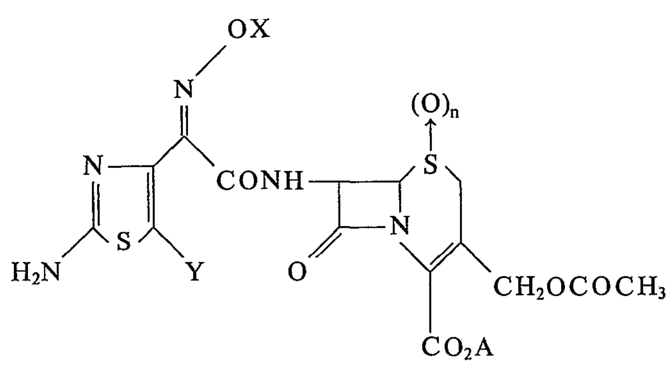

(I)

dans laquelle:
n est 0 ou 1 et X représente l'hydrogène, un radical alkyle ayant de 1 à 4 atomes de carbone, un radical carboxyméthyle, alcoxycarbonylméthyle ayant de 1 à 4 atomes de carbone dans le groupe alcoxy,

aminocarbonylméthyle ou cyanométhyle, les groupes carboxyméthyle, alcoxycarbonylméthyle, aminocarbonylméthyle et cyanométhyle pouvant également être encore substitués sur leur groupe méthylène par un groupe alkyle ayant de 1 à 4 atomes de carbone, Y représente un radical méthyle ou un halogène, A représente l'hydrogéne, un reste ester facilement séparable ou un cation physiologiquement acceptable et le groupe =N—OX est sous la forme syn.

2. Procédé de préparation de composés de formule générale I selon la revendication 1, caractérisé en ce qu'on fait réagir des lactames de formule générale II:

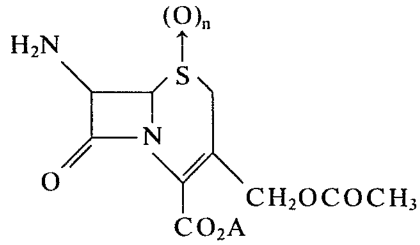

(II)

dans laquelle n et A ont les significations indiquées cidessus, avec un acide carboxylique de formule générale III:

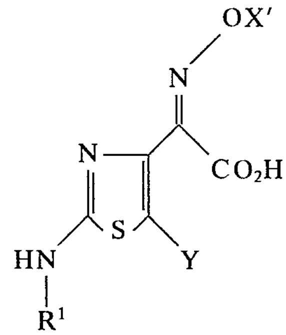

(III)

ou avec un dérivé activé de cet acide, où Y a la signification ci-dessus, $R^1$ représente l'hydrogène ou un groupe amino-protecteur connu dans la chimie des peptides et X' représente X, un groupe facilement séparable ou un groupe de formule:

$$—CH_2CO_2R^3$$

où $R^3$ représente un reste séparable dans des conditions modérées, pour obtenir un composé de formule générale IV:

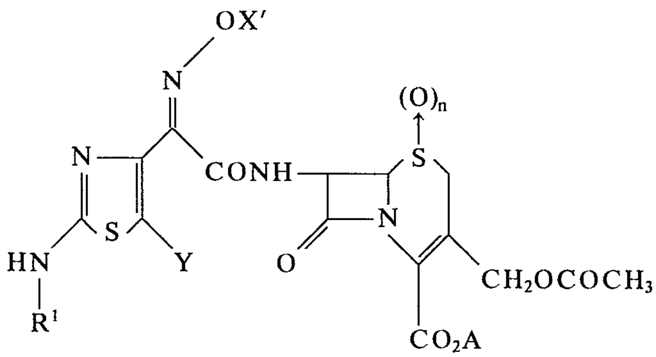

(IV)

dans laquelle $R^1$, X', Y et A ont les significations indiquées ci-dessus, et éventuellement on oxyde le produit ainsi obtenu sur le soufre du noyau céphème et/ou on sépare un reste $R^1$ représentant un groupe protecteur et/ou on transforme le groupe X' en le groupe X, dans la mesure où X' n'a pas la signification de X.

3. Compositions pharmaceutiques, caractérisées en ce qu'elles contiennent des composés de formule générale I selon la revendication 1.

4. Procédé pour la préparation de compositions pharmaceutiques, caractérisé en ce qu'on met sous une forme d'administration appropriée un composé de formule générale I selon la revendication 1, éventuellement associé avec des véhicules ou des diluants pharmaceutiques usuels.